# EUROPEAN PATENT SPECIFICATION

(11) **EP 1 973 893 B1**
(45) Date of publication and mention of the grant of the patent: **29.04.2015**
(21) Application number: 06841172.7
(22) Date of filing: 27.12.2006
(51) Int. Cl.: C07D 305/12

(54) **PROCESS FOR PREPARING CRYSTALLINE FORM II OF ORLISTAT**
VERFAHREN ZUR HERSTELLUNG DER KRISTALLINEN FORM II VON ORLISTAT
PROCEDE PERMETTANT DE PREPARER DE FORME II CRISTALLINE DE L'ORLISTAT

(30) Priority: 27.12.2005 EP 05028489
(43) Date of publication of application: 01.10.2008
(73) Proprietor: Krka Tovarna Zdravil, D.D., Novo Mesto, 8501 Novo Mesto (SI)
(72) Inventor: SIMONIC, Igor, 8351 Straza pri Novem mestu (SI); BENKIC, Primoz, 1000 Lubljana (SI); VAJS, Anamarija, 8000 Novo mesto (SI); KRAMAR, Andrejka, 8000 Novo mesto (SI); STIMAC, Anton, 1000 Ljubljana (SI)
(74) Representative: Becker Kurig Straus
(86) International application number: PCT/EP2006/012544
(87) International publication number: WO 2007/073937

(56) References cited:
- WO-A-03/047531
- WO-A-2005/026140
- US-A- 6 156 911
- PONS, J.-M.; KOCIENSKI, P.: "A Synthesis of (-)-Tetrahydrolipstatin" TETRAHEDRON LETT., vol. 30, no. 14, 1989, pages 1833-1836, XP002382427
- BARBIER, P. ET AL.: "STEREOSELECTIVE SYNTHESES OF TETRAHYDROLIPSTATIN AND OF AN ANALOGUE, POTENT PANCREATIC-LIPASE INHIBITORS CONTAINING A BETA-LACTONE MOIETY" HELVETICA CHIMICA ACTA, VERLAG HELVETICA CHIMICA ACTA. BASEL, CH, vol. 70, 1987, pages 1412-1488, XP002064047 ISSN: 0018-019X
- CHADHA, N. K. ET AL.: "SYNTHESIS OF TETRAHYDROLIPSTATIN" JOURNAL OF ORGANIC CHEMISTRY, AMERICAN CHEMICAL SOCIETY. EASTON, US, vol. 56, 1991, pages 4714-4718, XP002064049 ISSN: 0022-3263
- ROTE LISTE SERVICE GMBH (EDITOR): "Rote Liste 2004" 2004, EDITIO CANTOR VERLAG , AULENDORF , XP002382431 01003: Xenical 120 mg Hartkapseln.
- ANDERSON, N. G.: "Practical Process Research and Development" 2000, ACADEMIC PRESS , SAN DIEGO , XP002382432 Pages, 223-247, chapter 11: "Tools for Purifying the Product: Column Chromatography, Crystallization and Reslurrying"; in particular pages 228-232.
- BECKMANN, W.: "Seeding the Desired Polymorph: Background, Possiblities, Limitations, and Case Studies" ORGANIC PROCESS RESEARCH & DEVELOPMENT, vol. 4, 2000, pages 372-383, XP002382428
- European Pharmacopoeia, Edition 5.0, Chapter 5.4 "Residual Solvents".

## Description

The present invention relates to a process for preparing orlistat in crystalline form II. In particular, the present invention pertains to such a process, wherein a particular temperature profile is applied during the crystallization in specific organic solvents.

Orlistat is an agent providing a pancreatic lipase-inhibiting activity and is used for the preparation of medicaments for preventing and treating hyperlipaemia and obesity. Chemical names of Orlistat are (2S,3S,5S)-5-[(S)-2-formamido-4-methylvaleryloxy]-2-hexy-3-hydroxyhexadecanoic acid lactone or N-formyl-L-leucine-[2S-[2alpha(R*),3 beta]]-1-[(3-hexyl -4-oxo-2-oxetanyl)methyl]dodecyl ester. Orlistat exists in several crystalline forms. Analytic data and spectra characterizing the crystalline Form I and II of orlistat can be found in WO 2005/026140.

Syntheses for manufacturing orlistat and a possible precursor thereof, lipstatin, are already known in the art. Examples for suitable methods to manufacture lipstatin and orlistat are described for example in EP 0 129 748, EP 0 189 577, EP 1 028 115, WO 2004/003212, EP 0 803 576, WO 03/048335, EP 1 458 882, WO 2005/007639. Moreover, also several approaches for purifying orlistat have been previously described in the art. These approaches are however associated with significant drawbacks, and for example, provide insufficient yields, provide a product of insufficient purity or are associated with high costs or an insufficient reproducibility.

In particular, WO 2005/026140 describes a process for the preparation of crystalline Form II of orlistat, which comprises preparing a solution of orlistat in one or more ethers; and isolating the crystalline Form II from the solution thereof by the removal of the ether. As well known ether compounds may give rise to chemical hazards, either through the formation of ether peroxides or due to their low boiling point and high inflammability. Therefore, the use of ether as solvent is undesired and should be avoided.

WO 2003/047531 discloses a process for the preparation of orlistat crystalline form II which comprises the steps of mixing orlistat in hexane to form a mixture at a first temperature, lowering the temperature of the mixture sufficiently to precipitate and isolate crystalline orlistat form II.

The problem of the present invention is to provide a method which permits the production of orlistat in crystalline form II in good yields and in high crystalline quality. Additionally, such a method should be highly reproducible and reliable and help to ensure that crystals of the desired defined crystalline form will be obtained.

This problem has been solved by providing a process for preparing orlistat in a crystalline form II, said process comprising: providing a fluid consisting of orlistat and an organic solvent selected from pentane, hexane, heptane and octane and mixtures thereof and having a first temperature; lowering the temperature of said fluid from said first temperature to a second temperature, said second temperature being higher than the saturation temperature of orlistat in said fluid; adding orlistat seeding material having crystalline form II in order to obtain a crystallization mixture saturated or oversaturated at said second temperature; lowering the temperature of said crystallization mixture to a final temperature at a cooling rate of not more than 0.2 °C/min, isolating crystalline orlistat having said defined crystalline form, wherein orlistat seeding material having crystalline form II is obtainable by a process comprising the steps of:
(a) dissolving orlistat form I, amorphous form of orlistat or waxy form of orlistat in diisopropyl ether, wherein a volume of diisopropyl ether is from 2 to 10 times the weight of orlistat form I, amorphous form of orlistat or waxy form of orlistat; and
(b) removing diisopropyl ether by any of distillation, distillation under vacuum, evaporation, filtration, filtration under vacuum, decantation, and centrifugation to obtain orlistat seeding material having crystalline form II.

In the figures,

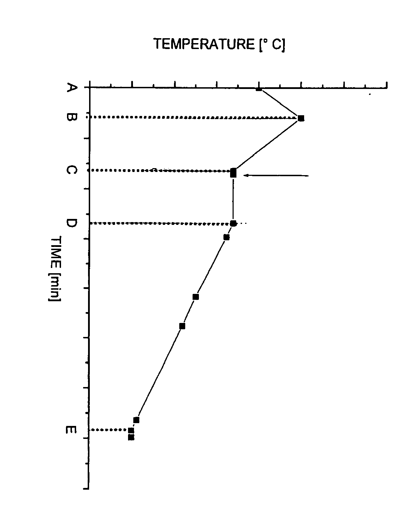

Figure 1 shows a graph describing a crystallization process of the present invention. At time "A" orlistate is added to a solvent, at time "B" the lowering of the temperature of the fluid from a first to a second temperature begins, at time "C" orlisat seeding material is added, at time "D" the lowering to a final temperature begins, at time "E" the final temperature is reached, and the ageing time is from "E" to "F".

The present invention provides a method which permits the production of orlistat of Form II via an addition of orlistat seeding material having a defined crystalline form. Additionally, the present invention teaches for the first time that for obtaining crystalline form II of orlistat a specific sequence of heating and cooling steps has to be performed and that the temperature of the crystallization mixture must be lowered at a cooling rate of not more than 0.2 °C/min. When observing the steps of the process according to the present invention, crystalline form II of orlistat of superior quality and in high yields may be obtained. Moreover, the present invention permits to omit an addition of hazardous solvents, such as in particular ethers, or the use of solvents which may not easily be removed from the desired crystalline product, such as water or alcohols, or could give rise to a formation of crystalline forms comprising crystal water.

In the first step of the method according to the present invention a fluid, in particular a solution, is prepared which consists of orlistat and an organic solvent selected from pentane, hexane, heptane and octane and mixtures thereof. This fluid can be prepared starting from any of the various forms of orlistat, for example starting from the amorphous or waxy form of orlistat, starting from the crystalline Forms I and II of orlistat, starting from a oily form of orlistat or starting from a crude product comprising orlistat. Orlistat can be prepared according to any desired method, for example according to a method described in EP 0 129 748, EP 0 189 577, EP 1 028 115, WO 2004/003212, EP 0 803 576, WO 03/048335, EP 1 458 882, WO 2005/007639, and in particular by means of a catalytic hydrogenation of lipstatin as described in the Examples of the present invention.

A particular advantage of the present invention is that said process provides the possibility to carry out the preparation of crystalline orlistat in a fluid of orlistat which is free of water or comprises only minor amounts of water remaining present in solvents dried according to standard procedures, such as drying over drying agents or distillation as described in standard laboratory manuals. Similarly, the process of the present invention helps to omit the presence of alcohols which often may not be easily removed during a drying step.

Advantageously, the fluid consisting of orlistat and the organic solvent is a solution. For specific applications, it should be however not excluded that non-dissolved material, in particular in minor amounts, such as e.g. of not more than 1 % by weight (when comparing the weight of the non-dissolved material in dry form to the weight of the dissolved orlistat in dry form) can be present or a clouding may be present in the fluid comprising orlistat and the organic solvent.

This fluid consisting of orlistat and the organic solvent is present at a first temperature. In the following step, the temperature of said fluid is lowered to a second temperature, which second temperature is higher than the saturation temperature of orlistat in said fluid, in particular in said solution. The saturation temperature of orlistat in a specific solvent or mixture can be determined on the basis of standard knowledge in the art and on the basis of standard experiments. Moreover, in the context of the present invention, it is considered that a temperature higher than the saturation temperature of orlistat in a particular fluid is present when after holding said fluid for 12 hours without stirring at the respective chosen temperature no spontaneous crystallization and precipitation of orlistat occurs.

Subsequently, orlistat seeding material having the crystalline form II is added at this second temperature and a solution or fluid saturated or over-saturated with respect to orlistat at said second temperature is obtained. In the context of the present invention, a fluid or solution saturated or over-saturated with respect to orlistat is considered to be present when additional solid orlistat (added e.g. in an amount of 100 mg to a solution or fluid of 1000 ml) remains optically detectable after holding said crystallization mixture for 12 hours without stirring at the respective chosen temperature.

As seeding material orlistat Form II is used which is obtainable by a process comprising the steps of: (a) dissolving orlistat form I, amorphous form of orlistat or waxy form of orlistat in diisopropyl ether, wherein a volume of diisopropyl ether is from 2 to 10 times the weight of orlistat form I, amorphous form of orlistat or waxy form of orlistat; and (b) removing diisopropyl ether by any of distillation, distillation under vacuum, evaporation, filtration, filtration under vacuum, decantation, and centrifugation to obtain orlistat seeding material having crystalline form II.

Analytic data and spectra characterizing the crystalline Form II of orlistat are known in the art and may be found in WO 2005/026140.

Advantageously, after the addition of the orlistat seeding material, the so obtained crystallization mixture is held for a period of time at said second temperature in order to permit that an ageing of crystals occurs. This additional period of time is advantageous for ensuring high quality and good yields of orlistat according to the present invention. Said additional period of time can take between 1 minute and 1 day, preferably between 10 minutes and 3 hours, more preferably between 15 minutes and 45 minutes.

In the next step, the temperature of the resulting crystallization mixture is lowered at a cooling rate of not more than 0.2 °C/min. A cooling step which is performed at this cooling rate is very important in order to obtain crystalline orlistat of high quality and in good yields and is important for ensuring that orlistat in the defined crystalline form corresponding to the seeding crystal will be obtained.

Particularly good results are obtained when the temperature of said crystallization mixture is lowered at a cooling rate of not more than 0.1 °C/min, preferably wherein the temperature of said crystallization mixture is lowered at a cooling rate of 0.02 to 0.07 °C/min, more preferably wherein the temperature of said crystallization mixture is lowered at a cooling rate of 0.03°C/min. In contrast, an immediate cooling to room temperature, to 0 °C or to an even lower temperature as usually performed in the art after having dissolved orlistat in a solvent, may give rise to devastating results and can give rise to orlistat of an undefined crystalline form, at least in a considerable number of crystallization experiments. The present invention thus largely increases the reliability and reproducibility of the crystallization process.

After having reached the final temperature and optionally having kept the crystallization mixture for an additional period of time allowing an ageing of the crystals (said additional period being for example up to additional 2 days, preferably up to additional 3 hours) at said temperature, the crystals will be separated from the crystallization mixture.

This separation of the orlistat crystals can be obtained by any separation process of the state of the art, for example by means of a filtration or a vacuum filtration. When desired, the crystals can be washed additionally with a small amount of the organic solvent(s) used in the process of the present invention or other appropriate organic solvent(s). In particular, it is advantageous, when the final temperature is in the range of - 30 °C to + 20 °C.

Optionally after the separation and isolation of orlistat a drying step can be performed. This drying step can be performed according to any suitable method known in the art, e.g. a drying procedure "under vacuum", i.e. at a pressure lower than the ambient pressure, and/or a drying procedure at room temperature or at a higher temperature.

The process of the present invention permits an access to orlistat in the crystalline Form II in a reproducible manner and in good yields.

Preferred hydrocarbons are hexane, heptane or octane. Particularly good results have been achieved when using heptane or octane, and especially when using heptane.

According to the general knowledge in the art, also mixtures of one or more of the hydrocarbon(s) can be used in a process according to the present invention.

Additional features and advantages of the present invention are described in and will become apparent from the following, non-limiting examples.

The pharmaceutical composition comprising orlistat in the crystalline Form II can be prepared through the dry or wet granulation procedure. To improve the flowability characteristic of the orlistat different diluents could be used: lactose, mannitol, sorbitol, microcrystalline cellulose and others. A part from the orlistat and diluents, the composition which is preferably in form of capsule, may also include other excipients and additives conventional in the art, like binders, solubilizing agents, lubricants, glidants and combinations thereof.

### Examples

### Example 1

### Hydrogenation of lipstatin

5.1 g of oily lipstatin (assay 92%) is dissolved in 102 ml of ethanol. The solution is stirred under argon for 5 minutes, then 440 mg of 5% Pd/C is added and the mixture is hydrogenated for 6 hours at ambient temperature and pressure. After hydrogenation, the resulting mixture is filtered and evaporated to the oily substance. 5.03 g of oil which contains 83% of orlistat is obtained.

### Example 2

### Preparation of crystals orlistat

2.78 g of oily orlistat obtained in Experiment 1 is mixed with 11 ml of heptane at room temperature and dissolved by heating to 30°C. The solution is then cooled to 22°C and seeded by 20 mg of orlistat form II. The mixture is then stirred for additional 0.5 hour and cooled in about 2 hours to 10°C. After additional 10 min of stirring, the solid is filtered off and dried in vacuum for 48 hours. 1.5 g (54% yield) of orlistat is obtained. The assay of product is 97%, the form of product is form II confirmed by IR spectra and XR diffractogram.

### Example 3

### Preparation of crystals orlistat

200 g of orlistat of polymorphic form II is mixed with 800 ml of heptane at room temperature and dissolved by heating to 32°C. The solution is then cooled to 22°C and seeded by I g of orlistat form II. The mixture is then stirred for I hour at 22°C and cooled in about 3 hours to 10°C. The solid is filtered off and dried in vacuum for 24 hours at temp 20 - 25°C. 184 g (92% yield) of orlistat is obtained. The assay of product is 97%, the form of product is form II confirmed by IR spectra and XR diffractogram.

### Example 4 (not according to the invention)

### Preparation of crystals orlistat

15 g of orlistat of polymorphic form I is mixed with 60 ml of heptane at room temperature and dissolved by heating to 30°C. The solution is then cooled to 22°C and seeded by 20 mg of orlistat form II. The mixture is then stirred for 1.5 hour at 22°C and cooled in about 4 hours to 15°C. The solid is filtered off and dried in vacuum for 24 hours at temp 20 - 25°C. 12.6 g (84% yield) of orlistat is obtained. The assay of product is 99%, the form of product is form II confirmed by IR spectra and XR diffractogram.

### Example 5

### Formulation containing orlistat prepared by granulation

| Ingredient | mg/capsule |
|---|---|
| Orlistat | 120.0 |
| Lactose anhydrous | 105.6 |
| Sodium lauryl sulfate | 7.2 |
| Talc | 7.2 |
| Total | 240.0 |

1. Orlistat and lactose anhydrous are mixed in a high share mixer and granulated with the ethanol.
2. Granules are dried at or below 30° C and passed through # 18 mesh screen.
3. Sodium lauryl sulphate and talc are added to the granulate and mixed.
4. Granules are filled in a hard gelatine capsule.

In the same example water or the mixture of water and ethanol can be used as a granulation liquid.

### Example 6

### Formulation containing orlistat prepared by dry process

| Ingredient | mg/capsule |
|---|---|
| Orlistat | 120.0 |
| Lactose anhydrous | 57.9 |
| Microcrystalline cellulose | 57.9 |
| Talc | 4.2 |
| Total | 240.0 |

1. Orlistat, lactose anhydrous, microcrystalline cellulose and talc are mixed in a high share mixer.
2. Prepared mixture is filled in a hard gelatine capsule.

## Claims

1. A process for preparing orlistat in a crystalline form II, said process comprising:
providing a fluid consisting of orlistat and an organic solvent selected from pentane, hexane, heptane and octane and mixtures thereof and having a first temperature;
lowering the temperature of said fluid from said first temperature to a second temperature, said second temperature being higher than the saturation temperature of orlistat in said fluid;
adding orlistat seeding material having crystalline form II in order to obtain a crystallization mixture saturated or oversaturated at said second temperature;
lowering the temperature of said crystallization mixture to a final temperature at a cooling rate of not more than 0.2 °C/min;
isolating crystalline orlistat having said defined crystalline form, wherein orlistat seeding material having crystalline form II is obtainable by a process comprising the steps of:
(a) dissolving orlistat form I, amorphous form of orlistat or waxy form of orlistat in diisopropyl ether, wherein a volume of diisopropyl ether is from 2 to 10 times the weight of orlistat form I, amorphous form of orlistat or waxy form of orlistat; and
(b) removing diisopropyl ether by any of distillation, distillation under vacuum, evaporation, filtration, filtration under vacuum, decantation, and centrifugation to obtain orlistat seeding material having crystalline form II.

2. The process according to claim 1 wherein the temperature of said crystallization mixture is lowered at a cooling rate of 0.02 to 0.07 °C/min.

3. The process according to claim 2, wherein the temperature of said crystallization mixture is lowered at a cooling rate of 0.03 °C/min.

4. The process according to any of the preceding claims, wherein the final temperature is in the range of - 30 °C to 20 °C.

## Patentansprüche

1. Verfahren zur Herstellung von Orlistat in kristalliner Form II, wobei das Verfahren umfasst:
Bereitstellen eines Fluids bestehend aus Orlistat und einem organischen Lösungsmittel ausgewählt unter Pentan, Hexan, Heptan und Oktan und Gemischen davon, das eine erste Temperatur aufweist,
Absenken der Temperatur des Fluids von der ersten Temperatur auf eine zweite Temperatur, wobei die zweite Temperatur über der Sättigungstemperatur von Orlistat in dem Fluid liegt,
Hinzufügen von Orlistat Impfmaterial mit kristalliner Form II, um ein Kristallisationsgemisch zu erhalten, das bei der zweiten Temperatur gesättigt oder übersättigt ist.
Absenken der Temperatur des Kristallisationsgemisches auf eine Endtemperatur mit einer Kühlrate von nicht mehr als 0,2°C/min.,
Isolieren von kristallinem Orlistat, das die definierte Kristallform aufweist, wobei das Orlistat Impfmaterial mit kristalliner Form II durch ein Verfahren erhalten wird umfassend die Schritte von:
(a) Lösen von Orlistat Form I, der amorphen Form von Orlistat oder der wachsartigen Form von Orlistat in Diisopropylether, wobei ein Volumen von Diisopropylether das Zwei- bis Zehnfache des Gewichts der Orlistat Form I, der amorphen Form von Orlistat oder der wachsartigen Form von Orlistat beträgt, sowie
(b) Entfernen des Diisopropylethers durch eines von Destillation, Destillation unter Vakuum, Evaporation, Filtration. Filtration unter Vakuum, Dekantieren und Zentrifugieren. um Orlistat Impfmaterial zu erhalten, das die kristalline Form II aufweist.

2. Verfahren nach Anspruch 1, wobei die Temperatur des Kristallisationsgemisches mit einer Kühlrate von 0,02 bis 0,07°C/min. abgesenkt wird.

3. Verfahren nach Anspruch 2. wobei die Temperatur des Kristallisationsgemisches mit einer Kühlrate von 0.03°C/min. abgesenkt wird.

4. Verfahren nach einem der vorstehenden Ansprüche, wobei die Endtemperatur in dem Bereich von -30°C bis 20°C liegt.

## Revendications

1. Procédé de préparation d'orlistat sous une forme cristalline II, ledit procédé comprenant :
la fourniture d'un fluide constitué d'orlistat et d'un solvant organique choisi parmi le pentane, l'hexane, l'heptane et l'octane et leurs mélanges et possédant une première température ;
l'abaissement de la température dudit fluide de ladite première température à une seconde température, ladite seconde température étant supérieure à la température de saturation de l'orlistat dans ledit fluide ;
l'ajout de matériau d'ensemencement d'orlistat possédant une forme cristalline II afin d'obtenir un mélange de cristallisation saturé ou sursaturé à ladite seconde température ;
l'abaissement de la température dudit mélange de cristallisation à une température finale à une vitesse de refroidissement ne dépassant pas 0,2 °C/min ;
l'isolement de l'orlistat cristallin possédant ladite forme cristalline définie, dans lequel le matériau d'ensemencement d'orlistat possédant la forme cristalline II peut être obtenu par un procédé comprenant les étapes consistant à :
(a) dissoudre une forme I d'orlistat, une forme amorphe d'orlistat ou une forme cireuse d'orlistat dans de l'éther di-isopropylique, dans lequel un volume d'éther di-isopropylique va de 2 à 10 fois le poids de forme I d'orlistat, de forme amorphe d'orlistat ou de forme cireuse d'orlistat ; et
(b) éliminer l'éther di-isopropylique par n'importe quel procédé parmi une distillation, une distillation sous vide, une évaporation, une filtration, une filtration sous vide, une décantation et une centrifugation de façon à obtenir un matériau d'ensemencement d'orlistat possédant la forme cristalline II.

2. Procédé selon la revendication 1,
dans lequel la température dudit mélange de cristallisation est abaissée à une vitesse de refroidissement de 0,02 à 0,07 °C/min.

3. Procédé selon la revendication 2, dans lequel la température dudit mélange de cristallisation est abaissée à une vitesse de refroidissement de 0,03 °C/min.

4. Procédé selon l'une quelconque des revendications précédentes, dans lequel la température finale est dans la plage de - 30 °C à 20 °C.
